(19) **Europäisches Patentamt**

**European Patent Office**

**Office européen des brevets**

(11) **EP 1 382 730 A1**

(12) **EUROPÄISCHE PATENTANMELDUNG**

(43) Veröffentlichungstag:
**21.01.2004 Patentblatt 2004/04**

(51) Int Cl.⁷: **D04H 1/46**, D04H 1/56

(21) Anmeldenummer: **02400033.3**

(22) Anmeldetag: **15.07.2002**

(84) Benannte Vertragsstaaten:
**AT BE BG CH CY CZ DE DK EE ES FI FR GB GR IE IT LI LU MC NL PT SE SK TR**
Benannte Erstreckungsstaaten:
**AL LT LV MK RO SI**

(71) Anmelder: **Paul Hartmann AG**
**89504 Heidenheim (DE)**

(72) Erfinder:
• **Mangold, Rainer Dr.**
**89542 Herbrechtingen (DE)**

• **Römpp, Angela**
**73119 Zell u.A. (DE)**
• **Michelmann, Jana**
**89522 Heidenheim (DE)**

(74) Vertreter: **Dreiss, Fuhlendorf, Steimle & Becker Patentanwälte,**
**Postfach 10 37 62**
**70032 Stuttgart (DE)**

(54) **Kosmetisches Wattepad**

(57) Die Erfindung betrifft ein kosmetisches Wattepad (2) mit einer verbesserten Abschminkwirkung, umfassend oder bestehend aus synthetischen Mikrostapelfasern einer Länge von wenigstens 7 mm.

M1:1

Fig 9

EP 1 382 730 A1

**Beschreibung**

Die Erfindung betrifft ein kosmetisches Wattepad, d. h. ein Wattepad zum Reinigen der Haut oder zum Abschminken, aber auch zum Auftragen kosmetischer Produkte, wie Cremes, auf die Haut.

Kosmetische Wattepads sind vielfach bekannt geworden. So offenbart EP 1 106 723 A1 ein Baumwoll-Wattepad mit beidseits wasserstrahlvernadelter Oberfläche. Das Baumwoll-Wattepad kann aber auch 0 - 30 % Kunstfasern, wie Viskosefasern, Polyesterfasern, Bikomponentenfasern, umfassen.

EP 0 836 842 B1 offenbart Wattepads und deren Herstellung, welche hydrophile Materialien umfassen, wie z. B. Baumwolle, Viskose oder Flachs, und/oder hydrophobe Materialien, wie z. B. Polyethylenterephtalat oder Polypropylen. Bevorzugt seien jedoch Wattepads, die zu 100 % aus Baumwolle bestehen oder solche, die zu 100 % aus Viskose bestehen oder aus wenigstens 25 % Viskose und der Rest Baumwolle oder aus wenigstens 25 % Baumwolle und der Rest Viskose oder aus 50 % Viskose und 50 % Polyethylenterephtalat oder aus 50 % Viskose und 50 % Polypropylen. Zur Faservliesverfestigung wird eine Wasserstrahlverfestigung vorgeschlagen.

EP 0 405 043 B1 offenbart ein dreischichtiges Wattepad, wobei die Schichten zu 100 % aus Baumwollkämmlingen bestehen. Die beiden äußeren Schichten sind stark verdichtet und an ihrer jeweiligen Sichtseite mit einem Waffelmuster versehen. Die drei Schichten werden dann zu einem sandwichartigen Aufbau zusammengeführt. Dabei sollen die Randpartien zumindest der äußeren Schichten miteinander verbunden werden.

Nach WO 00/76384 A1 soll ein Wattepad zu 100 % aus Baumwollfasern bestehen, wobei eine erste Schicht aus feinen Fasern einer Stärke von 0,7 - 1,75 denier (2 - 5 μg/pouce) und eine zweite Schicht aus demgegenüber stärkeren Fasern bestehen soll, welch letztere eine zum Abrieb bestimmte Oberfläche bilden soll.

US 4,100,324 beschreibt eine Fasermischung aus Holzzellstofffasern (Wood Pulp Fibers) und thermoplastischen Mikrofasern eines mittleren Faserdurchmessers von weniger als 10 μm. Dadurch dass diese Fasermischung quasi gleichzeitig mit dem Spinnen der Mikrofasern hergestellt wird, indem nämlich in den Bereich des Spinnkopfs für die Mikrofasern ein Luftstrom sowie ein Zellstoff-Faserstrom eingeströmt wird, entsteht eine Verbindung der Fasern und die Mikrofasern führen zu einer Fixierung der Zellstofffasern, und zwar in einem Zustand, in dem die Mikrofasern sich noch bei erhöhten Temperaturen in einem noch nicht gehärteten Zustand befinden. Die Mikrofasern wirken daher für die Holzzellstofffasern matrixbildend, wobei sie die Holzzellstofffasern gelenkig, also nicht starr einbinden, und zwar auch bei sehr geringen Gehalten an Mikrofasern bis herunter zu 1 Gew.-%. Es können zusätzlich weitere Fasern oder partikelförmiges Material, einschließlich synthetische Fasern wie Nylonfasern und natürliche Fasern wie Baumwolle, Flachs, Jute und Seide, eingebracht werden.

Durch die Herstellung der Fasermischung quasi in situ mit der Bildung der Mikrofasern im Schmelzblas-Prozess (melt blown-Verfahren) verlieren die Mikrofasern ihre Eigenständigkeit in dem Faserprodukt und werden aufgrund des Kontakts mit den Holzzellstoff-Fasern im noch erweichten Zustand verändert.

Der vorliegenden Erfindung liegt die Aufgabe zugrunde, ein kosmetisches Wattepad zu schaffen, welches sich einerseits sehr weich anfühlt, andererseits aber eine sehr hohe Reinigungs- oder Abschminkwirkung besitzt.

Diese Aufgabe wird erfindungsgemäß gelöst durch ein Wattepad umfassend oder bestehend aus synthetischen Mikrostapelfasern einer Länge von wenigstens 7 mm.

Wenn vorstehend von Mikrostapelfasern die Rede ist, so werden hierunter synthetische Fasern einer Faserstärke von ≤ 1 dtex verstanden. Der Begriff der Mikrostapelfasern bringt dabei zum Ausdruck, dass für die Herstellung der Faservlieslage des Wattepads zuvor in einem separaten Herstellungsverfahren gebildete Mikrofasern einer bestimmten Länge oder eines bestimmten Längenbereichs verwendet werden. Es hat sich gezeigt, dass synthetische Mikrostapelfasern einem Wattepad zu größerer Weichheit und damit zu einer hervorragenden Anfühlungswahrnehmung beim Benutzer zu verhelfen vermögen. Überraschenderweise ist damit aber nicht ein Rückgang der Reinigungs- oder Abschminkwirkung verbunden, sondern im Gegenteil weisen erfindungsgemäße Wattepads mit oder bestehend aus synthetischen Mikrostapelfasern einer Länge von wenigstens 7 mm, eine überlegene Reinigungs- und Abschminkwirkung auf.

Dies ist möglicherweise auf die aufgrund der Feinheit der Mikrostapelfasern zurückführbare große Oberfläche zurückzuführen, die zum Kontakt mit der zu reinigenden Hautoberfläche treten kann. Diese große Oberfläche begrenzt demzufolge auch eine riesige Anzahl von Mikrospalten und - öffnungen, welche zur Aufnahme von Unreinheiten, Hautschuppen oder Schminke dienen können.

Die Faserlänge der verwendbaren Mikrostapelfasern beträgt vorzugsweise 10 - 38 mm, insbesondere 15 - 32 mm.

Bei den Mikrostapelfasern kann es sich in weiterer Ausbildung der Erfindung um Polyester (PES) oder um Viskosefasern handeln. Die Oberfläche der Mikrostapelfasern sind vorzugsweise hydrophiliert. Auch kann die eine oder andere Oberfläche des Wattepads ein Prägemuster aufweisen. Auch eine Vliesverfestigung durch Wasserstrahlvernadeln oder den Zusatz wärmeschmelzbarer Bindefasern oder durch den Zusatz von chemischen Bindemitteln, wie z. B. wässrige Polymerdispersionen, z. B. Polyacrylate, Polyvinylacetate, Polyvinylalkohole, Latices oder Bindemittel auf Basis von Lösungsmitteln oder Polyurethane oder Streukleber/Schmelzklebepulver aus z. B. Polyamid, Polyethylen, Ethylenvinylacetat, Polyurethan oder Polyester, sind denkbar.

Das Flächengewicht des erfindungsgemäßen Kosmetikpads liegt vorzugsweise bei 40 - 300 g/m$^2$, insbesondere bei 60 - 250 g/m$^2$ und vorzugsweise bei 120 - 250 g/m$^2$ und besonders bevorzugtermaßen bei 150 - 250 g/m$^2$.

Insbesondere wenn das kosmetische Wattepad zur Flüssigkeitsaufnahme ausgebildet werden soll, wenn es sich also bspw. gut zur Aufnahme einer gesichtsreinigenden Lösung eignen soll oder zum Abschminken unter Verwendung eines einen hohen Flüssigkeitsanteil aufweisenden Abschminkmittels, erweist es sich als vorteilhaft, wenn das Wattepad zusätzlich bis 72 Gew.-% Baumwollfasern, insbesondere 15 - 65 Gew.-% und weiter insbesondere 50 - 65 Gew.-% Baumwollfasern enthält. Hierbei handelt es sich vorteilhafterweise um Baumwollkämmlinge. Das Wattepad kann solche Baumwollfasern umfassen, die zu wenigstens 0,2 Gew.-% mit einem Weichmacher beaufschlagt sind. Dieser Weichmacher kann ein Fettsäurekondensationsprodukt und/oder funktionelle Polydimethylsiloxane und/oder Polyethylene umfassen.

Des Weiteren erweist es sich als besonders vorteilhaft im Hinblick auf die Erreichung einer hohen inneren Festigkeit bei dem erfindungsgemäßen Wattepad, wenn zusätzlich wärmeschmelzbare Bindefasern, vorzugsweise zu 10 - 20 Gew.-%, enthalten sind, und mit diesen Bindefasern eine thermische Verfestigung des Wattepads durchgeführt wurde. Der Anteil wärmeschmelzbarer Bindefasern beträgt insbesondere 12 - 18 Gew.-% und weiter insbesondere 12 - 15 Gew.-% bezogen auf die Masse des Wattepads.

In weiterer Ausbildung der Erfindung kann es sich bei den Bindefasern um Mehrkomponentenfasern, insbesondere Bikomponenten-Fasern handeln mit einer höher schmelzenden Trägerkomponente und einer niedriger schmelzenden Komponente.

Die Mehrkomponentenfasern, insbesondere Bikomponentenfasern, haben in vorteilhafter Weise eine Faserstärke von 1,3 - 10 dtex, insbesondere von 1,3 - 3 dtex und eine Faserlänge von 3 bis 60 mm. Vorteilhafterweise kommen Kern/Mantel-Fasern oder Seite-an-Seite-Fasern zum Einsatz.

Es hat sich als vorteilhaft erwiesen, wenn Bikomponentenfasern mit einem Copolyester (CO-PES) als niedrig schmelzender Komponente und Polyester (PES) als höher schmelzender Komponente verwendet werden.

In weiterer Ausbildung der Erfindung von besonderer Bedeutung ist der Schmelzpunkt der wärmeschmelzbaren Bindefasern oder der niedrig schmelzenden Komponente (z.B. CO-PES)der Mehrkomponentenfasern geringer als der Schmelzpunkt der Mikrostapelfasern. Bspw. könnten Mikrostapelfasern aus einem Polyestermaterial verwendet werden mit einem Schmelzpunkt von etwa 256°C und CO-PES/PES-Bikomponentenfasern mit einem Schmelzpunkt der niedrig schmelzenden Komponente CO-PES von 110°C und der höher schmelzenden Komponente PES von 255°C. Solchenfalls könnte eine thermische Verfestigung des Faservlieses durchgeführt werden, ohne die höher schmelzende Komponente der Bikomponentenfasern und die Mikrostapelfasern thermisch zu verändern.

Ferner wird in Weiterbildung der Erfindung vorgeschlagen, dass eine Seite des Wattepads oberflächenrau ausgebildet ist, indem dort ein Schleifmittel in Form einer Beschichtung aufgebracht ist. Somit wird ein Wattepad geschaffen, welches eine als sehr weich empfundene Seite aufweist, die eine, wie vorausgehend erläutert, sehr hohe Reinigungswirkung besitzt, und eine raue Seite, die zum "Peeling", also zum Entfernen von abgestorbenen Hautschuppen, verwendet werden. Gleichzeitig kann hierbei ein angenehmer Massageeffekt erzielt werden.

Das Schleifmittel kann in vorteilhafter Weise aus einem thermoplastischen Schmelzklebepulver gebildet sein. Das Schmelzklebepulver kann als pulverförmige Komponente, bspw. mittels eines Präzisionsstreuers über ein Vibrationssieb auf die eine Seite des Wattepads aufgebracht und dort thermisch fixiert werden. Die thermische Fixierung erfolgt vorteilhafterweise durch einen Sinterprozess in einem Ofen. Hierbei wird das Schmelzklebepulver nicht aufgeschmolzen, sondern die Partikel werden unter Beibehaltung der die oberflächenraue Struktur bildenden Partikelform mit dem Wattepad durch einen Sinterprozess verbunden. Das hierfür vorteilhafterweise zu verwendete Schmelzklebepulver umfasst Polyethylen und/oder Polyamid und/oder Polyester. Es weist eine Körnung von 1 - 500 μm, insbesondere von 1 - 100 um, vorzugsweise von 1 - 65 μm auf. Es hat sich dabei als zweckmäßig und vorteilhaft erwiesen, wenn das Schleifmittel mit einem Flächengewicht von 5 - 50 g/m$^2$, insbesondere von 10 - 40 m$^2$ und vorzugsweise von 15 - 30 g/m$^2$ aufgebracht ist.

In weiterer Ausbildung des Erfindungsgedankens kann das erfindungsgemäße Wattepad herstellerseitig befeuchtet und im feuchten Zustand im Wesentlichen feuchtigkeitsdicht verpackt sein und dem Endverbraucher in dieser Form angeboten werden.

Auch Feuchttücher, also Feuchttuchvliese, aus dem vorausgehend erörterten erfindungsgemäßen Faservliesmaterial sind von der Erfindung erfasst. Auch feuchte und trockene Wattestäbchen können hieraus hergestellt werden.

Auch feuchte Wattepads oder Feuchttücher umfassen vorzugsweise Baumwollfasern, die zu wenigstens 0,2 Gew.-% mit einem Weichmacher, insbesondere der vorausgehend beschriebenen Art beaufschlagt sind. Vorteilhafterweise sind Feuchtpads mit einer Reinigungsemulsion in Form einer Öl-in-Wasser-Emulsion mit einer Viskosität von < 800 mPa befeuchtet. Die Öl-in-Wasser-Emulsion kann in einem Anteil von 0,2 - 0,5 Gew.-% einen Konservierungsstoff, bspw. Parabene oder Benzylalkohole, umfassen. Des Weiteren können 2 - 5 Gew.-% Pflanzenextraktanteil und 0,2 - 0,5 Gew.-% Parfumölanteil in der Öl-in-Wasser-Emulsion enthalten sein.

Zur Herstellung des erfindungsgemäßen Wattepads werden die in einem separaten Prozess hergestellten synthetischen Mikrostapelfasern zur Vliesbildung nach an sich üblichen Vliesbildungsverfahren abgelegt. Werden verschiedene Faserarten verwandt, so werden diese zuvor vorzugsweise in einem Luftstrom vermischt und dann abgelegt. Sind wärmeschmelzbare Bindefasern vorhanden, so kann in einem "Airthrough-Verfahren" mit genau einstellbarer Gastemperatur eine Thermofixierung des Vlieses vorgenommen werden, vorzugsweise ohne dass dabei die synthetischen Mikrostapelfasern thermisch und damit strukturell beeinflusst oder beeinträchtigt werden.

Alternativ oder auch zusätzlich hierzu kann eine Wasserstrahlvernadelung der Faservliesbahn vor oder nach der Thermofixierung vorgesehen werden. Unabhängig hiervon ist eine einseitige oder zweiseitige Prägekalandrierung möglich, wobei das Wattepad mit einem Oberflächenmuster ausgestattet werden kann.

Das erfindungsgemäße Wattepad ist vorzugsweise derart verdichtet, dass es eine Längsfestigkeit bzw. Höchstzugkraft in Längsrichtung (Maschinenrichtung) aufweist von 5 - 30 N/25mm, insbesondere 10 - 25 N/25 mm und vorzugsweise > 15 N/25 mm und eine Höchstzugkraft in Querrichtung (quer zur Maschinenrichtung) von 5 - 30 N/25mm, insbesondere 8 - 20 N/25mm und vorzugsweise > 10 N/25 mm auf. Diese Höchstzugkraft kann unter Verwendung einer genormten Zugprüfmaschine nach DIN 51221 nach der folgenden Prüfmethode ermittelt werden: Es werden aus dem zu prüfenden Wattepad, und zwar aus einem mittleren Bereich, Proben einer Einspannbreite von 25 mm und einer Einspannlänge von 30 mm genommen. Die in Klemmaufnahmen der normierten Zugprüfmaschine lotrecht eingespannten Proben werden dann mit einer Prüfgeschwindigkeit von 100 mm/min in der Ebene Ihrer Erstreckung auseinanderbewegt und dabei wird die in dieser Richtung wirkende Zugkraft gemessen. Unter der Höchstzugkraft wird diejenige maximale Kraft verstanden, bei der das Wattepad zerreist. Wenn zuvor höhere Kraftspitzen im Zuge der Dehnung gemessen werden, so stellen diese die Höchstzugkraft im Sinne dieser Prüfung dar. Man kann vorteilhafterweise bei Messungen der Längs- und der Querrichtung, welche der Maschinenrichtung bzw. der Richtung quer hierzu entspricht, verschiedene, insbesondere fünf Einzelmessungen vornehmen und deren Mittelwert berechnen.

Das erfindungsgemäße Wattepad weist ferner eine Dicke von vorzugsweise 0,5 - 4,5 mm auf, die unter einem spezifischen Messdruck von 0,5 kPa auf einer Tasterfläche von 25 cm$^2$ einer Probe des Prüflings ermittelt wurde. Das Prüfverfahren entspricht DIN EN ISO 9073-2 (Prüfverfahren für Vliesstoffe, Bestimmung der Dicke).

Des Weiteren kann das Absorptionsvermögen erfindungsgemäßer Wattepads bestimmt werden, und zwar wird hierfür entsprechend PH.EUR.1997, Monografie Verbandwatte aus Baumwolle, ein Test der Saugfähigkeit unter Bestimmung der Absinkdauer eines mit zu testenden Prüflingen befüllten Drahtkörbchens in einer Flüssigkeit ermittelt. Das hierzu verwendbare Drahtkörbchen ist ein zylindrischer Korb aus Kupferdraht mit einem Drahtdurchmesser von 0,4 mm. Die Höhe beträgt 80 mm, der Durchmesser 50 mm, die Maschenweite 15 - 20 mm und die Masse 2,7 +/- 0,3 g. Ferner findet ein Becherglas mit Durchmesser 11 - 12 cm Verwendung.

Zu testende Wattepads werden, bis eine Prüfmenge von 5 g vorhanden ist, in das Drahtkörbchen eingelegt. Zuvor wurde der Korb auf 0,01 g genau gewogen (M1) Die 5 g Probenmaterial bilden die Masse M2. Das Becherglas wird mit demineralisiertem Wasser bis zu einer Höhe von etwa 100 mm gefüllt und der gefüllte Korb wird aus einer Höhe von 10 mm auf das Wasser fallen gelassen. Mit einer Stopuhr wird die Zeit bis zum Absinken unter die Wasseroberfläche bestimmt. Unmittelbar nach der Bestimmung der Absinkdauer wird der Korb aus dem Wasser gehoben und zum Abtropfen 30 s lang in seiner Längsachse horizontal gehalten. Nach Ablauf der Abtropfzeit wird der Korb in ein tariertes Becherglas (M3) gegeben und auf 0,01 g genau gewogen (M4).

Das Wasserhaltevermögen ergibt sich

$$\text{in g/g} = \frac{M4 - (M2 + M3)}{M2 - M1}$$

Die Absinkdauer und das Wasserhaltevermögen werden als Mittelwert aus drei Bestimmungen angegeben. Für bevorzugte Wattepads beträgt die Absinkdauer maximal 15 sec und das Wasserhaltevermögen beträgt wenigstens 10 g/g. Dies lässt sich durch den Anteil absorbierender Fasern und/oder durch Zusatz von Hydrophilierungsmittel erreichen.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der Erfindung. In der Zeichnung zeigt:

Figuren 1 - 8      das Ergebnis eines Versuchs zur Bestimmung der Reinigungs- bzw. Abschminkwirkung bei Wattepads,

Figuren 9, 10      zwei Oberflächenmuster von Wattepads,

Figur 11      das Oberflächenmuster nach Fig. 10 im Detail.

Zwei bevorzugte Zusammensetzungen des erfindungsgemäßen Wattepads sind nachfolgend angegeben; sie sind mit PH und PH* gekennzeichnet.

| Faserart | PH | PH* |
|---|---|---|
| Mikrostapelfasern PES | 25 Gew.-% | 47 Gew.-% |
| Baumwollkämmlinge | 62 Gew.-% | 40 Gew.-% |
| Bikomponentenfasern CO-PES/PES | 13 Gew.-% | 13 Gew.-% |

Die Wattepads PH und PH* wurden dadurch erhalten, dass die zuvor hergestellten Mikrostapelfasern einer Faserstärke von 0,9 dtex und einer Faserlänge von ca. 18 mm mit Baumwollkämmlingen und den Bikomponentenfasern in einem Luftstrom in der jeweiligen gewichtsprozentualen Zusammensetzung gemischt wurden. Bei den Mikrostapelfasern handelt es sich um Fasern aus einem Polyester mit einem Schmelzpunkt von 256°C, und bei den Bikomponentenfasern handelt es sich um Kernmantelfasern mit einer höher schmelzenden Kernkomponente aus einem Polyester mit einem Schmelzpunkt bei 255°C und einem Mantel aus einem Copolyester (CO-PES) mit Schmelzpunkt bei 110°C. Die Länge dieser Bikomponentenfasern beträgt 51 mm; ihre Stärke 1,35 dtex.

Diese Wattepads wurden mit Vergleichsprodukten verglichen, wobei die nachfolgende Prüfung der Reinigungs- und Abschminkwirkung durchgeführt wurde: Hierbei wurde auf ein Substrat ein Make-up aufgebracht und unter standardisierten Bedingungen ermittelt, wie die jeweiligen Wattepads das Make-up von dem Substrat zu entfernen vermögen. Hierzu wird eine Zugprüfmaschine nach DIN 51221 Klasse 1 verwandt. Es wurde als Substrat "Ziegenglattleder beige zur Herstellung von Schuhen" verwandt. Auf ein 30 x 100 mm großes Stück dieses Ziegenglattleders wurde 0,1 g eines pflegenden Creme-Make-ups, nämlich der Marke Nivea Beauté Teint Natur Intensive, gleichmäßig aufgebracht und für zwei bis zweieinhalb Stunden trocknen gelassen.

Für die Versuchsdurchführung wird ein kreisrundes Löschpapier mit einem Durchmesser von 57 mm (Schleicher & Schüll, Nr. 860) in der Größe des zu testenden Wattepads mit einem Durchmesser von ebenfalls 57 mm ausgestanzt und mit 0,85 - 0,9 g demineralisiertem Wasser getränkt. Das runde Wattepad (Prüfling) (Durchmesser 57 mm, 25,5 cm$^2$) wird auf das getränkte Löschpapier gelegt und 10 s lang mit 1 kg belastet. Hierbei nimmt das Wattepad etwas Flüssigkeit auf. Das oberflächlich befeuchtete Wattepad wird nun auf das Ziegenleder in einer Zugrichtung vor die "geschminkte" Stelle gelegt. Ein zylindrisches Gewicht von 300 g und Durchmesser von 57 mm mit einer Öse wird mit der Zugvorrichtung der Zugprüfmaschine mittels eines Bindfadens verbunden. Das verbundene Gewicht wird mittels Doppelklebeband in exakter Überdeckung mit dem Wattepad fixiert. Wattepad und Gewicht werden dann mit einer Geschwindigkeit von 200 mm/min über die "geschminkte" Fläche gezogen. Es werden wenigstens fünf Abschminkversuche durchgeführt und jeweils die auf dem Ziegenleder verbleibende Menge an Make-up visuell oder mittels eines Farbmessgeräts (Chromameter) beurteilt. Als Prüflinge wurden die vorstehend erörterten Wattepads PH und PH* sowie ein aus 100 Gew.-% Baumwolle bestehendes Wattepad, PH 100 % Baumwolle, verwendet, wobei letzteres unter der Marke "Labell" im Handel erhältlich ist und wasserstrahlverfestigt ist. Weiter wurde ein im Handel erhältliches Wattepad der Marke "Demak' up Duo" sowie der Marke "Demak'up Supersoft" geprüft, welches ebenfalls aus 100 % Baumwolle besteht und wasserstrahlverfestigt ist. Wattepads der Marken "Nivea" und "Jean Carol" bestehen jeweils aus 100 % Baumwolle und sind ebenfalls wasserstrahlverfestigt. Ein beidseits geprägtes Wattepad der Marke "Hydra" wurde auch getestet.

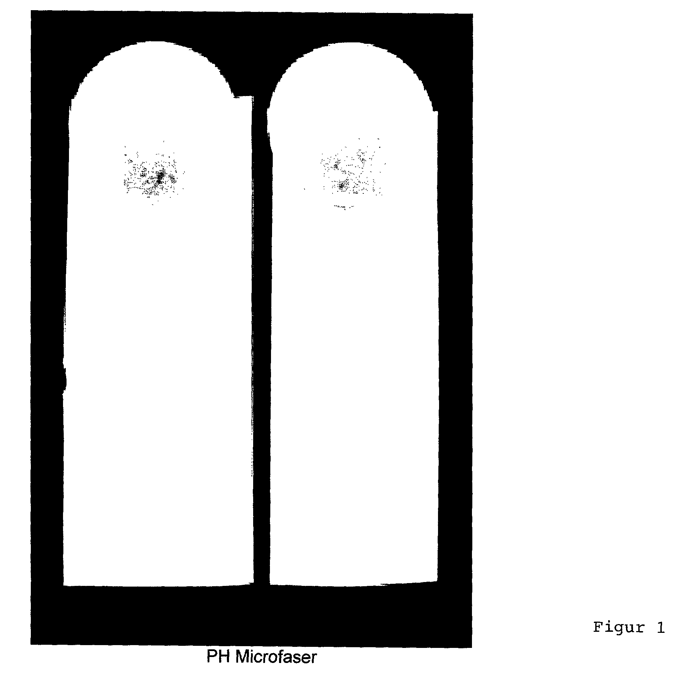

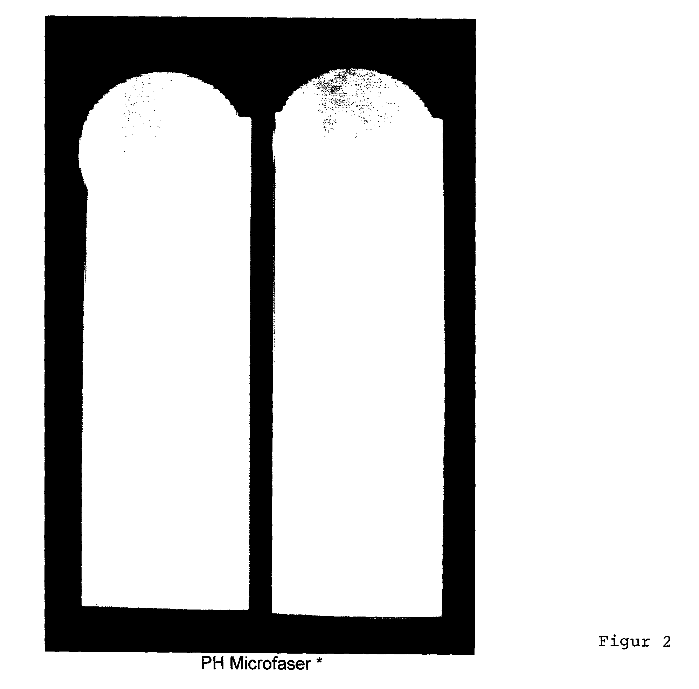

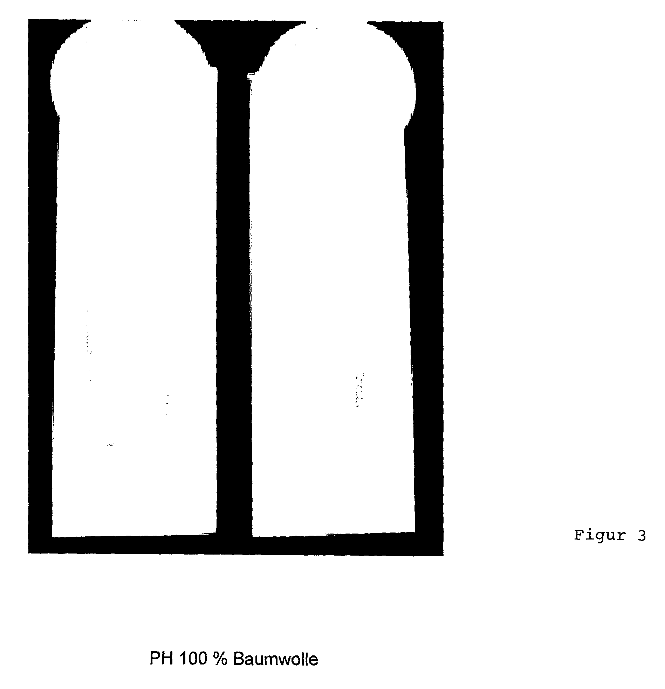

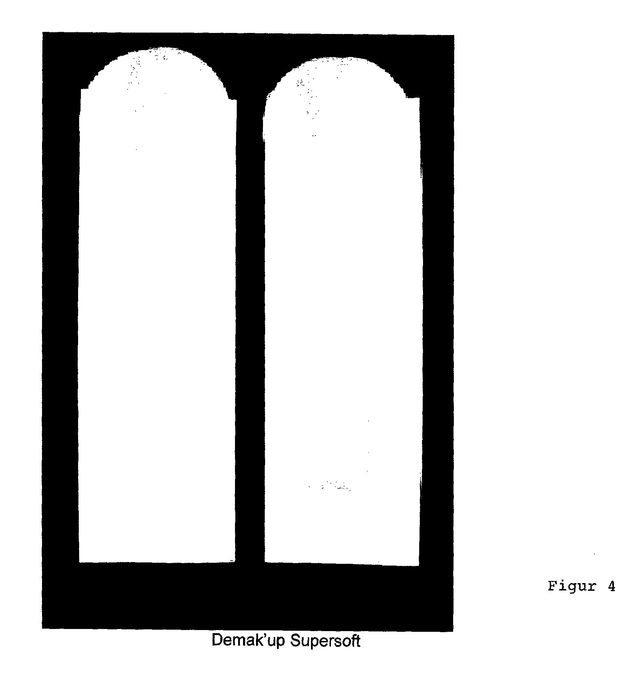

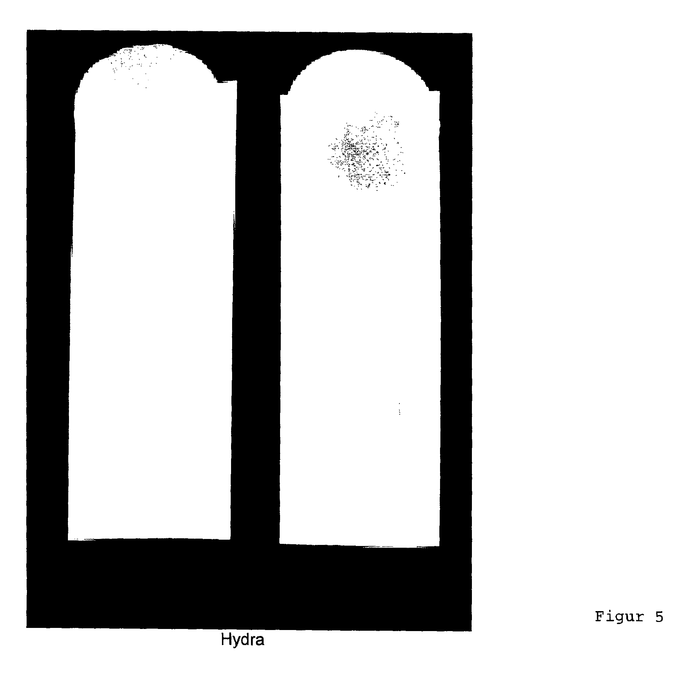

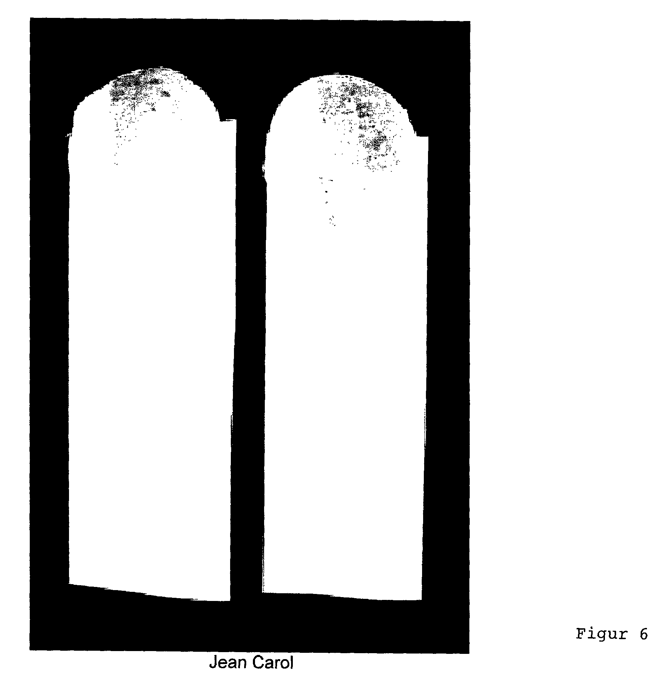

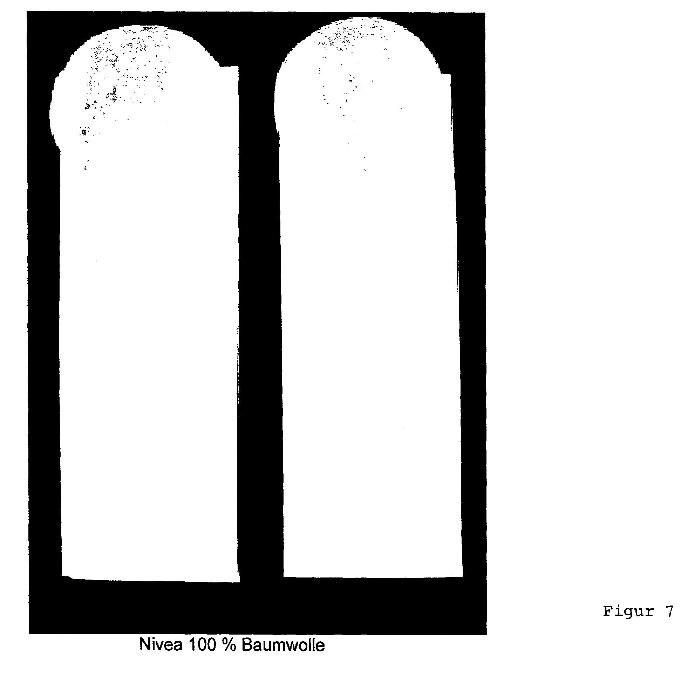

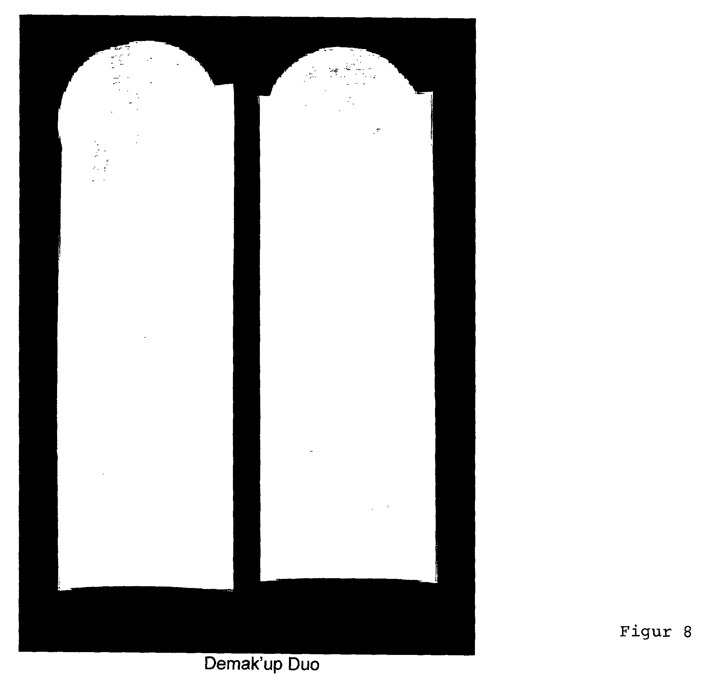

Die Figuren 1 bis 8 zeigen die fotografische Aufnahme je zweier Abschminkversuche, also das Bild, welches sich ergibt, wenn, wie vorstehend beschrieben, ein Wattepad in der beschriebenen Weise einmal über die "geschminkte" Fläche gezogen wurde. Das hierfür verwandte Wattepad wurde jeweils umgekehrt an die Ausgangsstelle des Abschminkversuchs auf das Substrat aus Ziegenleder positioniert und fotografiert.

Man erkennt, dass bei dem Produkt PH und PH* eine gegenüber den Vergleichsprodukten um ein Vielfaches effektivere Reinigungswirkung oder Abschminkwirkung erzielt wurde. Vergleicht man die erfindungsgemäßen Produkte PH und PH*, so führt die Erhöhung des Mikrofaseranteils zu einer Verbesserung der Abschminkwirkung.

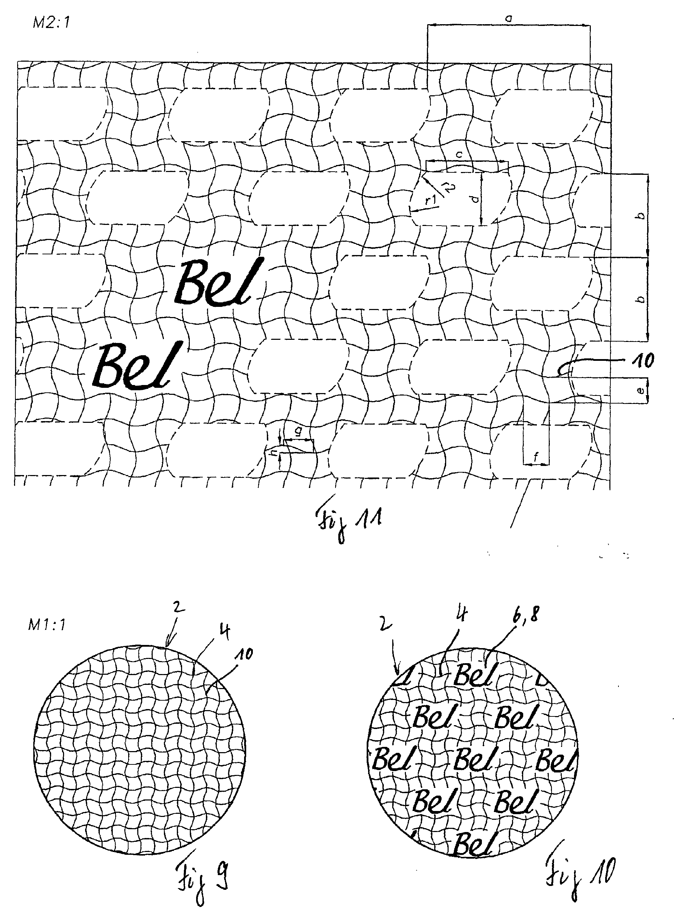

Figur 9 und 10 zeigen zwei Draufsichten auf ein erfindungsgemäßes Wattepad 2 mit unterschiedlichen Prägemustern 4, welche durch Prägekalandrieren des abgelegten Wattepads erzeugt wurden. Die Rillentiefe des Prägemusters 4 beträgt wenigstens 0,4 mm. Fig. 11 zeigt Einzelheiten des Prägemusters. Es umfasst eine wabenförmige oder waffelartige Strukturierung, die durch ein zweites Prägemuster 6 in Form eines Schriftzugs 8 unterbrochen ist. Die Abstände zwischen den Wabenlinien 10 betragen zwischen 2,0 und 5 mm, insbesondere zwischen 2,5 und 4,0 mm. Das zweite Prägemuster 6 nimmt eine Fläche von bis zu 15 x 10 mm (Abmessungen c x d) ein. Der Abstand zwischen den das jeweilige zweite Prägemuster 6 aufnehmenden Bereichen beträgt 3 - 10 mm (Abmessung b - d), insbesondere 4 - 6 mm in der einen Richtung bzw. 5 - 15 mm (Abmessung a - c), insbesondere 8 - 12 mm in der anderen Richtung.

Die wabenbildenden Prägelinien 10, welche das Prägemuster 4 bilden, sind wellenförmig mit einer halben Wellenlänge g von 2 - 8 mm, insbesondere 3 - 5 mm und mit einer Höhe h der Wellenlinie von 0,5 - 2 mm, insbesondere 0,8 - 1,2 mm.

**Patentansprüche**

1. Kosmetisches Wattepad umfassend oder bestehend aus synthetischen Mikrostapelfasern einer Länge von wenigstens 7 mm.

2. Kosmetisches Wattepad nach Anspruch 1, **dadurch gekennzeichnet, dass** es sich bei den Mikrostapelfasern um Polyester (PES)- oder um Viskosefasern handelt.

3. Kosmetisches Wattepad nach Anspruch 1 oder 2, **dadurch gekennzeichnet, dass** die Mikrostapelfasern an ihrer Oberfläche hydrophiliert sind.

4. Kosmetisches Wattepad nach Anspruch 1, 2 oder 3, **dadurch gekennzeichnet, dass** das Flächengewicht des Wattepads 40 - 300 g/m$^2$, insbesondere 60 - 250 g/m$^2$, insbesondere 120 - 250 g/m$^2$ und vorzugsweise 150 - 250 g/m$^2$ beträgt.

5. Kosmetisches Wattepad nach einem oder mehreren der vorstehenden Ansprüche, **dadurch gekennzeichnet, dass** der Anteil der Mikrostapelfasern 15 - 85 Gew.-%, insbesondere 15 - 65 Gew.-% und insbesondere 20 - 30 Gew.-% beträgt.

6. Kosmetisches Wattepad nach einem oder mehreren der vorstehenden Ansprüche, **dadurch gekennzeichnet, dass** zusätzlich bis 72 Gew.-%, insbesondere 15 - 65 Gew.-% und weiter insbesondere 50 - 65 Gew.-% Baumwollfasern enthalten sind.

7. Kosmetisches Wattepad nach Anspruch 6, **dadurch gekennzeichnet, dass** die Baumwollfasern Baumwollkämmlinge sind.

8. Kosmetisches Wattepad nach einem oder mehreren der vorstehenden Ansprüche, **dadurch gekennzeichnet, dass** zusätzlich wärmeschmelzbare Bindefasern enthalten sind, insbesondere in einem gewichtsprozentualen Anteil von 10 - 20 Gew.-%, insbesondere 12 - 18 Gew.-% und weiter insbesondere 12 - 15 Gew.-%.

9. Kosmetisches Wattepad nach Anspruch 8, **dadurch gekennzeichnet, dass** die Bindefasern Mehrkomponentenfasern, insbesondere Bikomponenten-Fasern sind.

10. Kosmetisches Wattepad nach Anspruch 9, **dadurch gekennzeichnet, dass** die Mehrkomponenten-Fasern eine Faserstärke von 1,3 - 10 dtex, insbesondere von 1,3 - 3,0 dtex und eine Faserlänge von 3 - 60 mm haben.

11. Kosmetisches Wattepad nach Anspruch 9 oder 10, **dadurch gekennzeichnet, dass** es sich bei den Bikomponentenfasern um Copolyester (CO-PES) / Polyester (PES)-Bikomponentenfasern handelt.

12. Kosmetisches Wattepad nach einem der Ansprüche 8 - 11, **dadurch gekennzeichnet, dass** der Schmelzpunkt der wärmeschmelzbaren Bindefasern oder der niedrig schmelzenden Komponente der Mehrkomponentenfasern geringer ist als der Schmelzpunkt der Mikrostapelfasern.

13. Kosmetisches Wattepad nach einem oder mehreren der vorstehenden Ansprüche, **dadurch gekennzeichnet, dass** die eine Seite des Wattepads oberflächenrauh ausgebildet ist, indem dort ein Schleifmittel aufgebracht ist.

14. Kosmetisches Wattepad nach Anspruch 13, **dadurch gekennzeichnet, dass** das Schleifmittel aus einem thermoplastischen Schmelzklebepulver gebildet ist, welches auf die eine Seite des Wattepads aufgesintert ist.

15. Kosmetisches Wattepad nach Anspruch 14, **dadurch gekennzeichnet, dass** das Schmelzklebepulver Polyethylen und/oder Polyamid und/oder Polyester umfasst.

16. Kosmetisches Wattepad nach Anspruch 13, 14 oder 15, **dadurch gekennzeichnet, dass** das Schleifmittel oder Schmelzklebepulver eine Körnung von 1 - 500 um, insbesondere 1 - 100 µm, vorzugsweise 1 - 65 µm aufweist.

17. Kosmetisches Wattepad nach einem der Ansprüche 13 - 16, **dadurch gekennzeichnet, dass** das Schleifmittel in einem Flächengewicht von 5 - 50 g/m$^2$, insbesondere 10 - 40 g/m$^2$, vorzugsweise 15 - 30 g/m$^2$ aufgebracht ist.

18. Kosmetisches Wattepad nach einem oder mehreren der vorstehenden Ansprüche, **dadurch gekennzeichnet, dass** es herstellerseitig befeuchtet und im feuchten Zustand im wesentlichen feuchtigkeitsdicht verpackt ist.

19. Kosmetisches Wattepad nach einem oder mehreren der vorstehenden Ansprüche, **dadurch gekennzeichnet, dass** es Baumwollfasern umfasst, die zu wenigstens 0,2 Gew.-% mit einem Weichmacher beaufschlagt sind.

20. Kosmetisches Wattepad nach Anspruch 19, **dadurch gekennzeichnet, dass** der Weichmacher ein Fettsäurekondensationsprodukt und/oder funktionelle Polydimethylsiloxane und/oder Polyethylene umfasst.

21. Kosmetisches Wattepad nach einem der Ansprüche 18 - 20, **dadurch gekennzeichnet, dass** es mit einer Öl-in-Wasser-Emulsion mit einer Viskosität von < 800 mPa befeuchtet ist.

22. Kosmetisches Wattepad nach Anspruch 21, **dadurch gekennzeichnet, dass** die Öl-in-Wasser-Emulsion einen Konservierungsstoff (Parabene, Benzylalkohol; 0,2 - 0,5 Gew.-%) umfasst.

23. Verwendung eines Wattepads nach einem oder mehreren der vorstehenden Ansprüche zum Reinigen und/oder Abschminken der Haut.

PH Microfaser

Figur 1

Figur 2

PH Microfaser *

Figur 3

PH 100 % Baumwolle

Figur 4

Demak'up Supersoft

Figur 5

Hydra

Figur 6

Jean Carol

Figur 7

Nivea 100 % Baumwolle

Figur 8

Demak'up Duo

M2:1

*fig 11*

M1:1

*fig 9*

*fig 10*

**Europäisches Patentamt**

## EUROPÄISCHER RECHERCHENBERICHT

Nummer der Anmeldung

EP 02 40 0033

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X,D | US 4 100 324 A (OSTERMEIER KURT W ET AL) 11. Juli 1978 (1978-07-11) * Beispiele 1,2,5-8 * * Spalte 7, Zeile 63 - Spalte 8, Zeile 9 * * Spalte 7, Zeile 24-44 * * Spalte 2, Zeile 52-54 * | 1-6,8,9, 18,23 | D04H1/46 D04H1/56 |
| Y | * Spalte 2, Zeile 29-38 * | 7,10,11, 13,17, 19-22 | |
| A | THE TEXTILE INSTITUTE: "TEXTILE TERMS AND DEFINITIONS" September 1988 (1988-09) , THE TEXTILE INSTITUTE , MANCHESTER XP002227582 * Seite 239, Absatz 7 * | 1 | |
| Y,D | EP 0 405 043 A (FLAWA SCHWEIZ VERBAND WATTEFAB) 2. Januar 1991 (1991-01-02) * Spalte 6, Zeile 23,24 * | 7 | |
| Y | EP 0 245 017 A (MINNESOTA MINING & MFG) 11. November 1987 (1987-11-11) | 10,11, 13,17 | RECHERCHIERTE SACHGEBIETE (Int.Cl.7) |
| A | * Seite 5, Zeile 11 - Seite 6, Zeile 23 * * Seite 7, Zeile 3-22 * | 12,14-16 | D04H A61F A61K |
| Y | US 4 559 157 A (SMITH JAMES A ET AL) 17. Dezember 1985 (1985-12-17) | 19-22 | A61L C11D |
| A | * Spalte 1, Zeile 6-10 * * Spalte 1, Zeile 41-50 * * Spalte 2, Zeile 28-58 * * Spalte 3, Zeile 7-25 * * Spalte 4, Zeile 4-14 * * Spalte 4, Zeile 58 - Spalte 5, Zeile 21 * | 18 | |

-/--

Der vorliegende Recherchenbericht wurde für alle Patentansprüche erstellt

| Recherchenort | Abschlußdatum der Recherche | Prüfer |
|---|---|---|
| DEN HAAG | 21. Februar 2003 | Joly, F |

EPO FORM 1503 03.82 (P04C03)

**Europäisches Patentamt**

## EUROPÄISCHER RECHERCHENBERICHT

Nummer der Anmeldung

EP 02 40 0033

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | EP 0 365 160 A (SCOTT PAPER CO) 25. April 1990 (1990-04-25) | 18-22 | |
| A | * Seite 2, Zeile 1-3 * <br> * Seite 3, Zeile 6,7 * <br> * Seite 3, Zeile 39-47; Beispiel 4 * <br> --- | 18 | |
| A | EP 0 926 288 A (UNI CHARM CORP) 30. Juni 1999 (1999-06-30) <br> * Spalte 2, Zeile 35-43 * <br> * Spalte 1, Zeile 47-53 * <br> --- | 1 | |
| A | EP 1 045 059 A (FREUDENBERG CARL FA) 18. Oktober 2000 (2000-10-18) <br> * Absatz '0019! * <br> * Absätze '0014!-'0016! * <br> * Absätze '0007!-'0009! * <br> --- | 1,2,4-6, 8-12 | |
| A | WO 99 24551 A (KIMBERLY CLARK CO) 20. Mai 1999 (1999-05-20) <br> * Seite 9, Zeile 9-12 * <br> * Seite 4, Zeile 24 - Seite 5, Zeile 18 * <br> * Seite 2, Zeile 2-4 * <br> --- | 1,4,5, 18,19,22 | RECHERCHIERTE SACHGEBIETE (Int.Cl.7) |
| A | EP 1 106 723 A (FORT JAMES FRANCE) 13. Juni 2001 (2001-06-13) <br> * Absätze '0080!-'0088! * <br> * Absätze '0035!-'0037! * <br> --- | 13-17 | |
| A | FR 2 795 100 A (FORT JAMES FRANCE) 22. Dezember 2000 (2000-12-22) <br> * Seite 2, Zeile 38 - Seite 3, Zeile 2; Anspruch 1 * <br> --- | 13-17 | |

-/--

Der vorliegende Recherchenbericht wurde für alle Patentansprüche erstellt

| Recherchenort | Abschlußdatum der Recherche | Prüfer |
|---|---|---|
| DEN HAAG | 21. Februar 2003 | Joly, F |

EPO FORM 1503 03 82 (P04C03)

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

Europäisches
Patentamt

# EUROPÄISCHER RECHERCHENBERICHT

Nummer der Anmeldung

EP 02 40 0033

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | FR 2 276 030 A (CIBA GEIGY AG) 23. Januar 1976 (1976-01-23) <br> * Seite 1, Zeile 21-29 * <br> * Seite 2, Zeile 21-31 * <br> * Seite 3, Zeile 19-30 * <br> * Seite 4, Zeile 16-22 * <br> * Seite 6, Zeile 19-22 * <br> * Seite 7, Zeile 4-6 * <br> * Seite 7, Zeile 39 – Seite 8, Zeile 4 * <br> * Seite 8, Zeile 21-24 * <br> * Seite 8, Zeile 35-38 * <br> * Ansprüche 1,4,15,16; Beispiele 16,17; Tabelle 4 * <br> --- | 13-20, 22,23 | |
| A | EP 0 826 811 A (AHLSTROM LYSTIL SA) 4. März 1998 (1998-03-04) <br> * Seite 2, Zeile 21 – Seite 3, Zeile 39 * <br> * Seite 4, Zeile 2-4 * <br> * Seite 5, Zeile 46-52 * <br> ----- | 13-17 | |
| | | | RECHERCHIERTE SACHGEBIETE (Int.Cl.7) |

Der vorliegende Recherchenbericht wurde für alle Patentansprüche erstellt

| Recherchenort | Abschlußdatum der Recherche | Prüfer |
|---|---|---|
| DEN HAAG | 21. Februar 2003 | Joly, F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03 82 (P04C03)

## ANHANG ZUM EUROPÄISCHEN RECHERCHENBERICHT
## ÜBER DIE EUROPÄISCHE PATENTANMELDUNG NR.

EP 02 40 0033

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

21-02-2003

| Im Recherchenbericht angeführtes Patentdokument | | | Datum der Veröffentlichung | Mitglied(er) der Patentfamilie | | | Datum der Veröffentlichung |
|---|---|---|---|---|---|---|---|
| US 4100324 | A | | 11-07-1978 | KEINE | | | |
| EP 0405043 | A | | 02-01-1991 | EP | 0405043 | A1 | 02-01-1991 |
| | | | | AT | 106007 | T | 15-06-1994 |
| | | | | DE | 58907716 | D1 | 30-06-1994 |
| EP 0245017 | A | | 11-11-1987 | US | 4769022 | A | 06-09-1988 |
| | | | | AU | 587692 | B2 | 24-08-1989 |
| | | | | AU | 7094187 | A | 05-11-1987 |
| | | | | BR | 8702033 | A | 09-02-1988 |
| | | | | CA | 1285111 | A1 | 25-06-1991 |
| | | | | DE | 3775610 | D1 | 13-02-1992 |
| | | | | EP | 0245017 | A1 | 11-11-1987 |
| | | | | ES | 2027692 | T3 | 16-06-1992 |
| | | | | JP | 62268517 | A | 21-11-1987 |
| | | | | KR | 9404703 | B1 | 27-05-1994 |
| | | | | MX | 160853 | A | 05-06-1990 |
| US 4559157 | A | | 17-12-1985 | KEINE | | | |
| EP 0365160 | A | | 25-04-1990 | US | 4904524 | A | 27-02-1990 |
| | | | | AU | 612100 | B2 | 27-06-1991 |
| | | | | AU | 4177789 | A | 26-04-1990 |
| | | | | CA | 1325560 | A1 | 28-12-1993 |
| | | | | DE | 68919991 | D1 | 26-01-1995 |
| | | | | DE | 68919991 | T2 | 18-05-1995 |
| | | | | DK | 514589 | A | 19-04-1990 |
| | | | | EP | 0365160 | A2 | 25-04-1990 |
| | | | | ES | 2065392 | T3 | 16-02-1995 |
| | | | | HK | 103995 | A | 07-07-1995 |
| | | | | JP | 2157217 | A | 18-06-1990 |
| | | | | JP | 2557987 | B2 | 27-11-1996 |
| | | | | KR | 139215 | B1 | 15-05-1998 |
| | | | | NO | 894131 | A ,B, | 19-04-1990 |
| EP 0926288 | A | | 30-06-1999 | CN | 1222597 | A | 14-07-1999 |
| | | | | JP | 11189959 | A | 13-07-1999 |
| | | | | US | 2002006760 | A1 | 17-01-2002 |
| | | | | AU | 9820398 | A | 15-07-1999 |
| | | | | BR | 9805841 | A | 14-12-1999 |
| | | | | EP | 0926288 | A1 | 30-06-1999 |
| | | | | SG | 71186 | A1 | 21-03-2000 |
| | | | | AU | 750350 | B2 | 18-07-2002 |
| EP 1045059 | A | | 18-10-2000 | DE | 19917275 | A1 | 19-10-2000 |
| | | | | AU | 740859 | B2 | 15-11-2001 |

EPO FORM P0461

Für nähere Einzelheiten zu diesem Anhang : siehe Amtsblatt des Europäischen Patentamts, Nr.12/82

**EP 1 382 730 A1**

## ANHANG ZUM EUROPÄISCHEN RECHERCHENBERICHT
## ÜBER DIE EUROPÄISCHE PATENTANMELDUNG NR.

EP 02 40 0033

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

21-02-2003

| Im Recherchenbericht angeführtes Patentdokument | | Datum der Veröffentlichung | Mitglied(er) der Patentfamilie | | Datum der Veröffentlichung |
|---|---|---|---|---|---|
| EP 1045059 | A | | AU | 2767100 A | 19-10-2000 |
| | | | CA | 2305114 A1 | 16-10-2000 |
| | | | CN | 1283718 A | 14-02-2001 |
| | | | EP | 1045059 A1 | 18-10-2000 |
| WO 9924551 | A | 20-05-1999 | AU | 734739 B2 | 21-06-2001 |
| | | | AU | 1393499 A | 31-05-1999 |
| | | | BR | 9813199 A | 16-10-2001 |
| | | | CA | 2309445 A1 | 20-05-1999 |
| | | | CN | 1285867 T | 28-02-2001 |
| | | | EP | 1030905 A1 | 30-08-2000 |
| | | | JP | 2001522935 T | 20-11-2001 |
| | | | PL | 342097 A1 | 21-05-2001 |
| | | | TR | 200001300 T2 | 21-11-2000 |
| | | | WO | 9924551 A1 | 20-05-1999 |
| | | | ZA | 9809994 A | 05-05-1999 |
| EP 1106723 | A | 13-06-2001 | EP | 1106723 A1 | 13-06-2001 |
| | | | AU | 2183901 A | 18-06-2001 |
| | | | BR | 0016189 A | 13-08-2002 |
| | | | DE | 29924071 U1 | 31-10-2001 |
| | | | DE | 1106723 T1 | 25-10-2001 |
| | | | WO | 0142548 A2 | 14-06-2001 |
| | | | NO | 20022673 A | 07-08-2002 |
| FR 2795100 | A | 22-12-2000 | FR | 2795100 A1 | 22-12-2000 |
| | | | EP | 1189531 A1 | 27-03-2002 |
| | | | EP | 1167605 A1 | 02-01-2002 |
| | | | WO | 0076384 A1 | 21-12-2000 |
| FR 2276030 | A | 23-01-1976 | DE | 2437165 A1 | 15-01-1976 |
| | | | FR | 2276030 A1 | 23-01-1976 |
| EP 0826811 | A | 04-03-1998 | FR | 2752248 A1 | 13-02-1998 |
| | | | DE | 69716689 D1 | 05-12-2002 |
| | | | EP | 0826811 A2 | 04-03-1998 |

EPO FORM P0461

Für nähere Einzelheiten zu diesem Anhang : siehe Amtsblatt des Europäischen Patentamts, Nr.12/82